(19) Europäisches Patentamt
European Patent Office
Office européen des brevets

(11) **EP 4 523 546 A1**

(12) **EUROPEAN PATENT APPLICATION**
published in accordance with Art. 153(4) EPC

(43) Date of publication:
**19.03.2025 Bulletin 2025/12**

(21) Application number: **23803646.1**

(22) Date of filing: **12.05.2023**

(51) International Patent Classification (IPC):
*A23J 3/34* (2006.01)     *A23J 3/14* (2006.01)
*A23L 33/185* (2016.01)     *C12P 21/06* (2006.01)

(52) Cooperative Patent Classification (CPC):
**A23J 3/14; A23J 3/34; A23L 33/185; C12P 21/06**

(86) International application number:
**PCT/JP2023/018009**

(87) International publication number:
**WO 2023/219172 (16.11.2023 Gazette 2023/46)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.05.2022 JP 2022079084
30.06.2022 JP 2022105971**

(71) Applicant: **Amano Enzyme Inc.
Nagoya-shi
Aichi 460-8630 (JP)**

(72) Inventor: **SAKAI, Kiyota
Kakamigahara-shi, Gifu 509-0109 (JP)**

(74) Representative: **Müller-Boré & Partner
Patentanwälte PartG mbB
Friedenheimer Brücke 21
80639 München (DE)**

(54) **METHOD FOR PRODUCING PROCESSED VEGETABLE PROTEIN-CONTAINING COMPOSITION**

(57) The purpose of the present invention is to provide a processing technology for increasing the free glutamic acid content of a protein hydrolysate. A processed plant protein-containing composition that is obtained by a method for producing a processed plant-containing composition which includes a step for treating a plant protein-containing composition with a proteolytic enzyme and a protein glutaminase has an increased free glutamic acid content.

**Description**

Technical Field

The present invention relates to a method for producing a processed plant protein-containing composition, and more specifically to a method for producing a plant protein-containing composition which is processed to increase a free glutamic acid content.

Background Art

In order to impart various properties such as improvement in digestion absorbability and reduction in allergen to a food product material containing a protein, processing of cleaving a protein using an endo-type protease is considered to be effective, and occurrent of bitterness at that time is a problem in technical development of food product processing.
This bitterness is caused by a bitter peptide generated by hydrolyzing a protein in a food product. A protein is one in which 100 to 1000 amino acids are bound in a single chain, and the chain is regularly folded and exists as a spherical mass. Hydrophilic amino acids are abundantly present on the outer side of the spherical mass, hydrophobic amino acids are abundantly present on the inner side, and when a protein is hydrolyzed, this massive structure is broken, and a portion containing a large amount of hydrophobic amino acids present on the inner side is cleaved to be exposed as a bitter peptide, which causes bitterness (NPL 1).
To address such a problem of bitterness, it has been proposed to treat a hydrolysate of the protein with glutaminase for γ-glutamylation of an amino acid exhibiting bitterness. Specifically, it has been confirmed that after a soybean protein (FUJIPRO F) is treated with a protease derived from Bacillus licheniformis to prepare a protein hydrolysate, this protein hydrolysate can be γ-glutamylated with a glutaminase derived from B. amyloliquefaciens, thereby reducing bitterness (NPL 2).

Citation List

Non Patent Literatures

NPL 1: Habibi-Najafi, M. B., and Lee, B. H., Bitterness in cheese. Crit. Rev. Food Sci. Nutr., 36, 397-411 (1996)
NPL 2: Hideyuki Suzuki, Yuko Nakafuji, Tomoki Tamura, New Preparation Method of Richness Taste Seasoning γ-Glutamylation of Protein Hydrolysate Obtained by Protease Treatment, Soy Protein Research Vol. 17 (2014), 42-45

Summary of Invention

Technical Problem

As described above, glutaminase treatment has been proposed as a treatment for reducing the bitterness of a predetermined protein hydrolysate, but since the glutaminase has narrow substrate specificity, the effect of improving the amount of glutamic acid released is limited, and there is still room for improvement.
Therefore, an object of the present invention is to provide a processing technology for increasing a free glutamic acid content of a protein hydrolysate. Solution to Problem
The present inventor has found that the free glutamic acid content is dramatically improved by treating a plant protein-containing composition with a proteolytic enzyme and a protein glutaminase. The present inventors have conducted further studies based on the findings, leading to the completion of the present invention.
That is, the present invention provides inventions of the following aspects.
Item 1. A method for producing a processed plant protein-containing composition, the production method including a step of treating a plant protein-containing composition with a proteolytic enzyme and a protein glutaminase.
Item 2. The production method according to item 1, in which the proteolytic enzyme is a protease and/or a peptidase.
Item 3. The production method according to item 1 or 2, in which the protease is used in an amount of 110 U or more per 1 g of the plant protein.
Item 4. The production method according to item 2 or 3, in which the protease is a filamentous fungus-derived protease.
Item 5. The production method according to any one of items 2 to 4, in which the protease is a protease derived from the genus Aspergillus and/or the genus Rhizopus.

Item 6. The production method according to any one of items 2 to 5, in which the protease is selected from the group consisting of proteases derived from Aspergillus oryzae, Aspergillus niger, Aspergillus melleus, and Rhizopus niveus.

Item 7. The production method according to item 2 or 3, in which the protease is a bacteria-derived protease.

Item 8. The production method according to item 7, in which the bacteria-derived protease is a protease derived from genus Bacillus and/or genus Geobacillus.

Item 9. The production method according to item 7 or 8, in which the bacteria-derived protease is selected from the group consisting of proteases derived from Bacillus stearothermophilus, Bacillus licheniformis, Bacillus amyloliquefaciens, and corresponding genus Geobacillus.

Item 10. The production method according to any one of items 2, 3, and 7 to 9, in which the proteolytic enzyme is a bacteria-derived protease and a filamentous fungus-derived peptidase.

Item 11. The production method according to any one of items 2 to 10, in which the peptidase is a peptidase derived from the genus Rhizopus and/or the genus Aspergillus

Item 12. The production method according to any one of items 1 to 11, in which the plant protein is a protein of a plant selected from the group consisting of beans, cereals, nuts, and seeds.

Item 13. The production method according to any one of items 1 to 12, in which the plant protein-containing composition is a liquid.

Item 14. The production method according to any one of items 1 to 12, in which the plant protein-containing composition is a textured plant protein material swollen with water.

Item 15. An agent of increasing a free glutamic acid content of a plant protein-containing composition, the agent including a proteolytic enzyme and a protein glutaminase.

Item 16. A plant protein-containing food or drink product obtained by the production method according to any one of items 1 to 14.

Advantageous Effects of Invention

According to the present invention, there is provided a processing technology for increasing a free glutamic acid content in a protein hydrolysate.

Description of Embodiments

1. Method for Producing Processed Plant Protein-Containing Composition

A method for producing a processed plant protein-containing composition of the present invention includes a step of treating a plant protein-containing composition with a proteolytic enzyme and a protein glutaminase (enzyme treatment step). Since the free glutamic acid content in the plant protein-containing composition can be improved (increased) by the enzyme treatment step, a plant protein-containing composition, which is processed to increase the free glutamic acid content, is obtained. Hereinafter, the method for producing a processed plant protein-containing composition of the present invention will be specifically described.

1-1. Plant Protein-Containing Composition

The plant protein-containing composition used in the present invention contains a plant protein and water, and is typically a composition for food and drink. There is no particular restriction on the aspect of the plant protein-containing composition, and examples thereof include an aspect having fluidity, such as a liquid, a slurry, or a paste, (hereinafter, these plant protein-containing compositions of the aspect having fluidity are also collectively referred to as "liquid and the like"), and a solid.

Specific examples of the plant protein-containing composition include, as one prepared from an untextured protein material, (i) a liquid and the like obtained by dispersing, in water, dry powder of a plant protein material (specifically, at least one of a plant organ of a plant protein-derived plant, and a material obtained by, for example, removing at least a part of components other than the protein from the plant organ to increase the content of the protein is exemplified; the same applies hereinafter); (ii) a liquid and the like obtained by crushing and dispersing a plant protein material in water, and removing an insoluble matter derived from a skin or the like of a plant material by any means such as centrifugation, filtration, filtration bag, or sieve, as necessary; (iii) a liquid and the like obtained by, for example, removing components other than the plant protein from the liquid and the like of the above (i) or (ii) to increase the content of the protein; and (iv) a liquid and the like obtained by mixing dry powder prepared from the liquid and the like of any one of the above (i) to (iii) with water, and include, as one prepared from a textured protein material, (v) a textured plant protein material swollen with water.

Note that, the textured plant protein material used in preparation of the specific example of the above (v) is generally a food product material known as an alternative meat (pseudo meat). Typical examples of the textured plant protein material include a material in which a raw material mixture containing a plant protein and water is extruded with an extruder or the like and dried or frozen to be textured like meat. Note that, in the present invention, the "meat" imitated by the textured plant protein material means a muscle of an animal that is edible, and when described as "meat", the "meat" is used in the sense of including not only muscles of mammals and birds but also fish and shellfish.

Examples of the shape of the textured plant protein material include a granular shape and a fibrous shape. Examples of the granular shape include massive shapes having various sizes such as a small grain type (mince), a large grain type, and a block type (the size increases in the order of the small grain type, the large grain shape, and the block type); and flat shapes of various sizes such as a flake type, a fillet type, and a slice type (the size increases in the order of the flake type, the fillet type, and the slice type).

More specific examples of the textured plant protein material include a granular plant protein and a fibrous plant protein. Both the granular plant protein and the fibrous plant protein refer to those defined in "Japanese Agricultural Standards of plant protein". However, the textured plant protein material used in the present invention is not limited to the granular plant protein and the fibrous plant protein defined above as long as it is a material textured like meat as described above.

Regarding the textured plant protein material that can be used in the present invention, the kind of the plant protein, the characteristics other than the content ratio of the plant protein (for example, properties, moisture content, grain size, product temperature, raw materials other than food additives, food additives, chewiness, water retentivity, foreign matters, and content amount), and the measurement method thereof can conform to the characteristics and the measurement method defined in "Japanese Agricultural Standards of plant protein".

The content of the plant protein contained in the textured plant protein material (based on the weight in a dry state of the textured plant protein material) is not particularly limited, and is, for example, 20 wt% or more, 25 wt% or more, or 30 wt% or more. From the viewpoint of further enhancing the effect of increasing the free glutamic acid content, the content is preferably 35 wt% or more, more preferably 40 wt% or more, and further preferably 45 wt% or more. The upper limit of the content range is not particularly limited, and is, for example, 90 wt% or less, preferably 80 wt% or less, more preferably 70 wt% or less, even more preferably 60 wt% or less, and further more preferably 55 wt% or less.

Preferred examples of the liquid plant protein-containing composition include plant milk.

Note that, in the following description, the "the content of the plant protein material" in the plant protein-containing composition refers to, in the case of a plant protein-containing composition prepared using an untextured plant protein material, a proportion occupied by the dry weight of the component constituting the above-described plant organ contained in the plant protein-containing composition, and refers to, in the case of a plant protein-containing composition prepared using a textured plant protein material, a proportion occupied by the dry weight of the textured plant protein material. For example, when the plant protein-containing composition is a liquid and the like composed only of a component derived from a plant organ and water as in the specific examples of (i) to (iv) described above, the "content of the plant protein material" refers to a proportion occupied by the dry weight of the liquid and the like. When the plant protein-containing composition is a liquid and the like containing the specific examples of (i) to (iv) described above and an additive, the "content of the plant protein material" refers to a proportion occupied by the dry weight of a portion obtained by removing the additive from the liquid and the like. When the plant protein-containing composition is a swollen product composed only of a textured plant protein material and water as in the specific example of (v) described above, the "content of the plant protein material" refers to a proportion occupied by the dry weight of the composition. When the plant protein-containing composition is a swollen product containing the specific example of (v) described above and an additive, the "content of the plant protein material" refers to a proportion occupied by the dry weight of a portion obtained by removing the additive from the swollen product.

The plant protein is not particularly limited, and examples thereof include proteins (natural proteins) contained in beans such as soybean, pea, lentil bean, chickpea, black bean, fava bean, mung bean, lupine bean, and haricot bean; cereals such as wheat, barley, oat, sorghum, rice, rye, buckwheat, Japanese barnyard millet, foxtail millet, teff, corn, and potato; nuts such as almond, coconut, peanut, cashew nut, hazelnut, pecan nut, macadamia nut, pistachio, walnut, brazil nut, pili nut, chestnut, sesame, and pine nut; and seeds such as hemp seed (industrial hemp), chia seed, quinoa, Amaranthus, canary seed, and linseed; chemically partially decomposed proteins of the above-described proteins by an acid, an alkali, or the like; and chemically modified protein by various reagents and synthetic peptides.

In the present invention, one kind of the plant protein may be used alone, or a plurality of kinds thereof may be used in combination. From the viewpoint of further increasing the free glutamic acid content, proteins of beans are preferable, proteins of soybean, pea, lentil bean, chickpea, fava bean, mung bean, and lupine bean are more preferable, and proteins of soybean and pea are further preferable.

The content of the plant protein in the plant protein-containing composition is not particularly limited, and in the case of preparing the plant protein-containing composition from an untextured protein material, the content is, for example, 0.01 to 50 wt%, 0.05 to 40 wt%, or 0.1 to 30 wt%, preferably 0.25 to 25 wt%, or 0.35 to 15 wt%, and more preferably 0.45 to 10 wt% or 0.6 to 9 wt%, and in the case of preparing the plant protein-containing composition from a textured protein material, the content is, for example, 1 to 70 wt%, preferably 5 to 50 wt%, more preferably 10 to 30 wt%, and further preferably 15 to 25 wt%.

The content of the plant protein material in the plant protein-containing composition is also not particularly limited, and in the case of preparing the plant protein-containing composition from an untextured protein material, the content is, for example, 0.05 to 50 wt%, 0.1 to 40 wt%, or 0.5 to 30 wt%, preferably 1 to 25 wt% or 3 to 20 wt%, and more preferably 5 to 15 wt% or 7 to 12 wt%, and in the case of preparing the plant protein-containing composition from a textured protein material, the content is, for example, 20 to 60 wt%, preferably 30 to 50 wt%, and more preferably 35 to 45 wt%.

Note that, when the plant protein contained in the plant protein-containing composition is a protein of a plant having a large amount of starchy material like cereals (preferably, barley, sorghum, or rye), such a plant protein-containing composition is preferably pre-treated with an amylase. Typical examples of the amylase include $\alpha$-amylase. The $\alpha$-amylase is not particularly limited, and examples thereof include $\alpha$-amylases derived from the genus Aspergillus (such as Aspergillus oryzae and Aspergillus niger), the genus Bacillus (such as Bacillus amyloliquefaciens, Bacillus subtilis, and Bacillus licheniformis), an $\alpha$-amylase derived from the genus Bacillus is preferable, and an $\alpha$-amylase derived from Bacillus amyloliquefaciens species is more preferable.

The amount of the $\alpha$-amylase used per 1 g of the plant protein-containing composition is, for example, 0.5 to 100 U, 1 to 50 U, 2 to 25 U, or 3 to 10 U, and preferably 4 to 8 U.

For the activity of the $\alpha$-amylase, the amount of enzyme that reduces the color of potato starch with iodine by 10% per minute is defined as 1 unit (1 U).

1-2. Proteolytic Enzyme

As the proteolytic enzyme, an enzyme capable of decomposing a peptide chain can be used without particular limitation, and typical examples thereof include a protease and/or a peptidase.

1-2-1. Protease

In the present invention, the protease refers to an endo-type peptidase. The origin of the protease is not particularly limited, and examples thereof include a filamentous fungus-derived protease and a bacteria-derived protease.

The filamentous fungus-derived protease is not particularly limited, and examples thereof include proteases derived from the genera Aspergillus, Rhizopus, Mucor, Neurospora, Penicillium, Rhizomucor, and Sclerotinia.

Specific examples of the protease derived from the genus Aspergillus include proteases derived from Aspergillus oryzae, Aspergillus niger, Aspergillus melleus, Aspergillus japonicus, Aspergillus awamori, Aspergillus kawachii, Aspergillus sojae, Aspergillus tamarii, Aspergillus foetidus, Aspergillus fumigatus, Aspergillus nidulans, Aspergillus aculeatus, Aspergillus candidus, Aspergillus flavus, Aspergillus saitoi, Aspergillus inuii, Aspergillus glaucus, Aspergillus caesiellus, Aspergillus clavatus, Aspergillus deflectus, Aspergillus fischerianus, Aspergillus parasiticus, Aspergillus penicilloides, Aspergillus restrictus, Aspergillus sydowii, Aspergillus terreus, Aspergillus ustus, and Aspergillus versicolor.

Specific examples of the protease derived from the genus Rhizopus include proteases derived from Rhizopus chinensis, Rhizopus delemar, Rhizopus niveus, and Rhizopus oryzae.

Examples of the bacteria-derived protease include proteases derived from the genera Bacillus and Geobacillus.

Specific examples of the protease derived from the genus Bacillus (or Geobacillus) include proteases derived from Bacillus amyloliquefaciens, Bacillus cereus, Bacillus clausii, Bacillus intermedius, Bacillus lentus, Bacillus licheniformis, Bacillus stearothermophilus, Bacillus subtilis, Bacillus thermoproteolyticus, and corresponding genus Geobacillus.

In the present invention, one kind of the protease may be used alone, or a plurality of kinds thereof may be used in combination. Among these proteases, from the viewpoint of further enhancing the effect of increasing the free glutamic acid content, a filamentous fungus-derived protease and/or a bacteria-derived protease is preferable.

Among the above-described filamentous fungus-derived proteases, from the viewpoint of further enhancing the effect of increasing the free glutamic acid content, a protease derived from the genus Aspergillus and a protease derived from the genus Rhizopus are preferable. Among the above-described proteases derived from the genus Aspergillus, from the viewpoint of further enhancing the effect of increasing the free glutamic acid content, a protease derived from Aspergillus oryzae, a protease derived from Aspergillus niger, and a protease derived from Aspergillus melleus are preferable, and among the above-described proteases derived from the genus Rhizopus, from the viewpoint of further enhancing the effect of increasing the free glutamic acid content, a protease derived from Rhizopus niveus is preferable.

Among the above-described bacteria-derived proteases, from the viewpoint of further enhancing the effect of increasing the free glutamic acid content, a protease derived from Bacillus stearothermophilus, a protease derived from Bacillus licheniformis, a protease derived from Bacillus amyloliquefaciens, and a protease derived from corresponding genus Geobacillus are preferable, a protease derived from Geobacillus stearothermophilus, a protease derived from Bacillus amyloliquefaciens, and a protease derived from Bacillus licheniformis are more preferable, and a protease derived from Bacillus licheniformis is further preferable.

The amount of the protease used is not particularly limited, and the amount used per 1 g of the plant protein material is, for example, 110 U or more. From the viewpoint of further enhancing the effect of increasing the free glutamic acid content, the amount used is preferably 200 U or more, 350 U or more, or 500 U or more, more preferably 700 U or more or 900 U or more, further preferably 1000 U or more, and even more preferably 1100 U or more. The upper limit of range of the amount of the protease used per 1 g of the plant protein material is not particularly limited, and is, for example, 300000 U or less, 150000 U or less, 100000 U or less, 75000 U or less, 50000 U or less, 40000 U or less, 30000 U or less, 25000 U or less, 20000 U or less, 15000 U or less, or 10000 U or less.

The amount of the protease used per 1 g of the plant protein is, for example, 110 U or more, and from the viewpoint of further enhancing the effect of increasing the free glutamic acid content, is preferably 200 U or more, 350 U or more, or 500 U or more, more preferably 750 U or more or 1000 U or more, further preferably 1200 U or more, and even more preferably 1300 U or more or 1400 U or more. The upper limit of the range of the amount of the protease used per 1 g of the plant protein is not particularly limited, and is, for example, 600000 U or less, 300000 U or less, 150000 U or less, 100000 U or less, 75000 U or less, 60000 U or less, 50000 U or less, 40000 U or less, 30000 U or less, 25000 U or less, or 20000 U or less.

The protease activity is measured using casein as a substrate by the Folin method. That is, the protease activity is determined in such a manner that an enzymatic reaction is performed using casein as a substrate at a pH set according to the optimum pH of a protease to be measured by a conventional method, and an amount of an enzyme which causes an increase in colored materials by Folin's reagent corresponding to 1 μg of tyrosine per minute is defined as 1 unit (1 U).

1-2-2. Peptidase

In the present invention, the peptidase refers to an exo-type peptidase. The origin of the peptidase is not particularly limited, and examples thereof include a filamentous fungus-derived peptidase, an actinomycete-derived peptidase, and a bacteria-derived peptidase.

The filamentous fungus-derived peptidase is not particularly limited, and examples thereof include peptidases derived from the genera Rhizopus and Aspergillus. The actinomycete-derived peptidase is not particularly limited, and examples thereof include peptidases derived from the genus Streptomyces. Specific examples of the peptidase derived from the genus Rhizopus include peptidases derived from Rhizopus oryzae, and specific examples of the peptidase derived from the genus Aspergillus include a peptidase derived from Aspergillus oryzae.

The bacteria-derived peptidase is not particularly limited, and examples thereof include peptidases derived from the genera Bacillus, Geobacillus, Lactobacillus, and Lactococcus.

In the present invention, one kind of the peptidase may be used alone, or a plurality of kinds thereof may be used in combination. Among the above-described peptidases, from the viewpoint of further enhancing the effect of increasing the free glutamic acid content, a filamentous fungus-derived peptidase is preferable, peptidases derived from the genera Rhizopus (particularly, Rhizopus oryzae) and Aspergillus (particularly, Aspergillus oryzae) are more preferable, and a peptidase derived from the genus Rhizopus (particularly, Rhizopus oryzae) is further preferable.

The amount of the peptidase used is not particularly limited, and the amount of the peptidase used per 1 g of the plant protein material is, for example, 0.001 U or more in any of the cases of preparing the plant protein-containing composition from an untextured protein material and a textured protein material. From the viewpoint of further enhancing the effect of increasing the free glutamic acid content, the amount of the peptidase used is preferably 0.005 U or more, 0.01 U or more, or 0.015 U or more, more preferably 0.025 U or more, 0.05 U or more, 0.075 U or more, or 0.1 U or more, further preferably 0.13 U or more, 0.15 U or more, 0.18 U or more, 0.2 U or more, or 0.23 U or more, and even more preferably 0.25 U or more or 0.27 U or more. In the case of preparing the plant protein-containing composition from a textured protein material, the amount of the peptidase used per 1 g of the plant protein material is preferably 0.5 U or more or 1 U or more, more preferably 2.5 U or more, 5 U or more, or 7.5 U or more, further preferably 10 U or more, 15 U or more, or 20 U or more, even more preferably 25 U or more or 30 U or more, and further more preferably 35 U or more or 40 U or more.

The upper limit of the range of the amount of the peptidase used per 1 g of the plant protein material is not particularly limited, and is, for example, 500 U or less, 300 U or less, 200 U or less, 100 U or less, 75 U or less, 50 U or less, or 45 U or less in any of the cases of preparing the plant protein-containing composition from an untextured protein material and a textured protein material. In the case of preparing the plant protein-containing composition from a textured protein material, the upper limit of the range of the amount of the peptidase used per 1 g of the plant protein material is, for example, 500 U or less, preferably 400 U or less, or 300 U or less, more preferably 200 U or less, or 100 U or less, further preferably 80 U or less, 70 U or less, or 60 U or less, and even more preferably 55 U or less or 50 U or less.

The amount of the peptidase used per 1 g of the plant protein is, for example, 0.001 U or more in any of the cases of preparing the plant protein-containing composition from an untextured protein material and a textured protein material, and from the viewpoint of further enhancing the effect of increasing the free glutamic acid content, is preferably 0.005 U or more, 0.01 U or more, or 0.015 U or more, more preferably 0.025 U or more or 0.05 U or more, further preferably 0.075 U or more or 0.1 U or more, even more preferably 0.13 U or more, 0.15 U or more, 0.18 U or more, 0.2 U or more, or 0.23 U or more, further more preferably 0.25 U or more or 0.27 U or more, and particularly preferably 0.3 U or more or 0.33 U or more. In the case of preparing the plant protein-containing composition from a textured protein material, the amount of the peptidase used per 1 g of the plant protein is preferably 1 U or more or 2.5 U or more, more preferably 5 U or more or 10 U or more, further preferably 20 U or more, 30 U or more, or 40 U or more, even more preferably 50 U or more, 60 U or more, or 70 U or more, and even further preferably 75 U or more or 80 U or more.

The upper limit of the range of the amount of the peptidase used per 1 g of the plant protein is not particularly limited, and is, for example, 1000 U or less, 900 U or less, 800 U or less, 700 U or less, 600 U or less, 500 U or less, 300 U or less, 200 U or less, 100 U or less, or 90 U or less in any of the cases of preparing the plant protein-containing composition from an untextured protein material and a textured protein material. In the case of preparing the plant protein-containing composition from a textured protein material, the upper limit of the range of the amount of the peptidase used per 1 g of the plant protein is, for example, 1000 U or less, 800 U or less, 600 U or less, 500 U or less, 400 U or less, 300 U or less, 200 U or less, 150 U or less, 100 U or less, or 90 U or less.

The peptidase activity is measured by a method based on the ninth edition of Japan's Specifications and Standards for Food Additives using L-leucyl-p-nitroanilide hydrochloride as a substrate, and specifically, when an enzyme reaction is performed using L-leucyl-p-nitroanilide hydrochloride as a substrate by a usual method, the activity that generates 1 $\mu$mol of p-nitroaniline per minute is defined as 1 unit (1 U).

1-3. Protein Glutaminase

The type, origin, and the like of the protein glutaminase used in the present invention are not particularly limited as long as it is an enzyme that exhibits an action of decomposing an amide group-containing side chain of a glutamine residue without cleaving peptide bonds and crosslinking the protein. Examples of the protein glutaminase include a protein glutaminase derived from the genus Chryseobacterium, Flavobacterium, Empedobacter, Sphingobacterium, Aureobacterium, or Myroides, which is disclosed in Japanese Patent Laid-open Publication Nos. 2000-50887 and 2001-218590, and WO 2006/075772 A. These protein glutaminases may be used singly or in combination of a plurality of kinds thereof.

Among these protein glutaminases, from the viewpoint of further enhancing the effect of increasing the free glutamic acid content, a protein glutaminase derived from the genus Chryseobacterium is preferable, and a protein glutaminase derived from Chryseobacterium proteolyticum species is more preferable.

The protein glutaminase can be prepared from a culture solution of microorganisms from which the protein glutaminase is derived. Specific examples of the preparation method include a method of recovering a protein glutaminase from a culture solution or a bacterial cell of the above-mentioned microorganism. For example, in the case of using a microorganism that secrets a protein glutaminase, an enzyme can be separated and/or purified after recovering bacterial cells from the culture solution in advance by filtration, a centrifugal treatment, or the like, as necessary. In the case of using a microorganism that does not secret a protein glutaminase, an enzyme can be separated and/or purified after recovering bacterial cells from the culture solution in advance as necessary and then disrupting the bacterial cells by a pressurization treatment, an ultrasonic treatment, or the like to expose an enzyme. As an enzyme separation and/or purification method, a known protein separation and/or purification method can be used without particular limitation, and examples thereof include a centrifugal separation method, a UF concentration method, a salting-out method, and various chromatography methods using an ion-exchange resin or the like. The separated and/or purified enzyme can be powdered by a drying method such as freeze-drying or reduced-pressure drying, and can also be powdered using an appropriate excipient and/or drying aid in the drying method. The separated and/or purified enzyme can also be liquefied by adding an appropriate additive and performing filtration sterilization.

The amount of the protein glutaminase used is not particularly limited, and the amount of the protein glutaminase used per 1 g of the plant protein material is, for example, 0.01 U or more in any of the cases of preparing the plant protein-containing composition from an untextured protein material and a textured protein material. From the viewpoint of further enhancing the effect of increasing the free glutamic acid content, the amount of the protein glutaminase used per 1 g of the plant protein material is preferably 0.5 U or more, more preferably 0.75 U or more, further preferably 1.0 U or more, and even more preferably 1.2 U or more. In the case of preparing the plant protein-containing composition from a textured protein material, the amount of the protein glutaminase used per 1 g of the plant protein material is preferably 0.01 U or more, more preferably 0.1 U or more, or 0.25 U or more, further preferably 0.5 U or more or 0.8 U or more, and even more preferably 1 U or more or 1.2 U or more.

The upper limit of the range of the amount of the protein glutaminase used per 1 g of the plant protein material is not particularly limited, and is, for example, 200 U or less, 150 U or less, 100 U or less, or 50 U or less, preferably 30 U or less, more preferably 15 U or less, or 10 U or less, and further preferably 7.5 U or less, 5 U or less, 2.5 U or less, or 2 U or less in any of the cases of preparing the plant protein-containing composition from an untextured protein material and a textured protein material. In the case of preparing the plant protein-containing composition from a textured protein material, the upper limit of the range of the amount of the protein glutaminase used per 1 g of the plant protein material is, for example, 200 U or less, 150 U or less, 120 U or less, 100 U or less, 80 U or less, 60 U or less, 40 U or less, 30 U or less, 20 U or less, 10 U or less, 7.5 U or less, 5 U or less, 2.5 U or less, or 2 U or less.

The amount of the protein glutaminase used per 1 g of the plant protein is, for example, 0.15 U or more in any of the cases of preparing the plant protein-containing composition from an untextured protein material and a textured protein material. From the viewpoint of further enhancing the effect of increasing the free glutamic acid content, the amount of the protein glutaminase used per 1 g of the plant protein is preferably 0.5 U or more, more preferably 0.75 U or more, further preferably 1.0 U or more, and even more preferably 1.2 U or more. In the case of preparing the plant protein-containing composition from a textured protein material, the amount of the protein glutaminase used per 1 g of the plant protein is preferably 0.1 U or more or 0.25 U or more, more preferably 0.5 U or more or 0.75 U or more, further preferably 1 U or more or 1.5 U or more, and even more preferably 2 U or more or 2.5 U or more.

The upper limit of the range of the amount of the protein glutaminase used per 1 g of the plant protein is not particularly limited, and is, for example, 400 U or less, 300 U or less, 200 U or less, 150 U or less, 100 U or less, or 50 U or less, preferably 30 U or less, 25 U or less, or 20 U or less, more preferably 15 U or less, or 10 U or less, and further preferably 7.5 U or less, 5 U or less, or 2.5 U or less in any of the cases of preparing the plant protein-containing composition from an untextured protein material and a textured protein material. In the case of preparing the plant protein-containing composition from a textured protein material, the upper limit of the range of the amount of the protein glutaminase used per 1 g of the plant protein is, for example, 400 U or less, 300 U or less, 200 U or less, 100 U or less, 50 U or less, 25 U or less, 20 U or less, 10 U or less, or 5 U or less.

The ratio of the proteolytic enzyme and the protein glutaminase used is determined based on the above amount of each enzyme used, and from the viewpoint of further enhancing the effect of increasing the free glutamic acid content, the following used ratio is preferable.

The amount of the protein glutaminase used per 1 U of the protease is preferably 0.005 mU or more, more preferably 0.01 mU or more or 0.02 mU or more, further preferably 0.03 mU or more, 0.04 mU or more, or 0.045 mU or more, and even more preferably 0.05 mU or more, 0.075 mU or more, 0.1 mU or more, or 0.15 mU or more. The upper limit of the range of the amount of the protein glutaminase used per 1 U of the protease is, for example, 15 mU or less, and from the viewpoint of further enhancing the effect of increasing the free glutamic acid content, is preferably 12 mU or less or 10 mU or less, more preferably 8 mU or less or 6 mU or less, further preferably 5 mU or less or 4 mU or less, and even more preferably 3 mU or less, 2 mU or less, or 1.5 mU or less.

The amount of the protein glutaminase used per 1 U of the peptidase is preferably 0.001 U or more, more preferably 0.005 U or more, or 0.01 U or more, further preferably 0.015 U or more, 0.02 U or more, or 0.025 U or more, and even more preferably 0.03 U or more in any of the cases of preparing the plant protein-containing composition from an untextured protein material and a textured protein material. In the case of preparing the plant protein-containing composition from a textured protein material, the amount of the protein glutaminase used per 1 U of the peptidase is more preferably 0.005 U or more, further preferably 0.01 U or more or 0.02 U or more, and even more preferably 0.025 U or more or 0.03 U or more. The upper limit of the range of the amount of the protein glutaminase used per 1 U of the peptidase is, for example, 100 U or less in any of the cases of preparing the plant protein-containing composition from an untextured protein material and a textured protein material, and from the viewpoint of further enhancing the effect of increasing the free glutamic acid content, is preferably 75 U or less, 50 U or less, or 40 U or less, more preferably 30 U or less or 20 U or less, further preferably 15 U or less or 10 U or less, and even more preferably 7 U or less, 6 U or less, or 5.5 U or less. In the case of preparing the plant protein-containing composition from a textured protein material, the upper limit of the range of the amount of the protein glutaminase used per 1 U of the peptidase is preferably 3 U or less, more preferably 2 U or less, or 1 U or less, further preferably 0.5 U or less or 0.1 U or less, and even more preferably 0.075 U or less or 0.05 U or less.

For the activity of the protein glutaminase, benzyloxycarbonyl-L-glutaminylglycine (Z-Gln-Gly) is used as a substrate, and the amount of enzyme that liberates 1 $\mu$mol of ammonia per minute is defined as 1 unit (1 U).

### 1-4. Reaction Conditions, etc.

In the enzyme treatment step, a reaction of liberating glutamic acid in the plant protein-containing composition is advanced by the treatment.

The order of the actions of the proteolytic enzyme and the protein glutaminase is not particularly limited, and the enzymes may be sequentially made to act in any order, or both the enzymes may be simultaneously made to act, and preferably, both the enzymes can be simultaneously made to act.

The conditions (such as temperature, pH, and time) for the treatment with the proteolytic enzyme and the protein glutaminase are appropriately selected according to the intended effect of increasing the free glutamic acid content.

The temperature at which the treatment with the proteolytic enzyme and the protein glutaminase is performed is not particularly limited, and can be appropriately determined by those skilled in the art according to the optimum temperature of the enzyme to be used and/or the thermal properties of the plant protein-containing composition, and the like, and is, for example, 30°C to 80°C, preferably 40°C to 70°C, and more preferably 40°C to 60°C.

The pH of a reaction system in which the treatment with the proteolytic enzyme and the protein glutaminase is performed is also not particularly limited, and can be appropriately determined by those skilled in the art according to the optimum pH of the enzyme to be used and/or the pH property of the plant protein-containing composition, and the like, and the pH at 25°C is, for example, 2 to 9, preferably 3 to 8, more preferably 5 to 7, and further preferably 6 to 7.

The time for performing the treatment with the proteolytic enzyme and the protein glutaminase is, for example, 0.5 to 96 hours, preferably 1 to 72 hours, and further preferably 1 to 30 hours.

1-5. Other Steps

The production method of the present invention can include any other steps in addition to the above-described enzyme treatment step. Specific examples of the other steps include, as a step performed before the enzyme treatment step, a step of preparing the plant protein-containing composition, and include, as a step performed after the enzyme treatment step, a deactivation step of the proteolytic enzyme and the protein glutaminase, a filtration step of a processed plant protein-containing composition, and a drying step of the processed plant protein-containing composition. Examples of the method in the drying step include freeze drying, vacuum drying, and spray drying.

2. Plant Protein-Containing Food or Drink Product

A plant protein-containing food or drink product of the present invention is a processed plant protein-containing composition obtained by "1. Method for Producing Processed Plant Protein-Containing Composition" or a prepared product using the processed plant protein-containing composition as a food or drink product material. In the plant protein-containing food or drink product of the present invention, the free glutamic acid content is increased.

When the plant protein-containing food or drink product of the present invention is a prepared product of the processed plant protein-containing composition, as a preparation method for obtaining the prepared product, any preparation method performed for obtaining a food or drink product alternative to a general animal protein prepared product can be used. Specific examples of the preparation method include seasoning, food additive blending, mixing of other food or drink product materials, heating, fermentation, molding, freezing, and/or firing.

The aspect of the plant protein-containing food or drink product of the present invention is not particularly limited. The aspect when the plant protein-containing food or drink product of the present invention is a processed plant protein-containing composition is determined according to the aspect of the plant protein-containing composition, the presence or absence of the drying step, and the like in the above-described production method, and examples thereof include aspects having fluidity, such as a liquid, a slurry, and a paste, and a solid, and specific examples of the solid include a powder state, a fine particle state, a granular state, a textured state swollen with moisture, and a dried textured state. When the plant protein-containing food or drink product of the present invention is a prepared product of the processed plant protein-containing composition, as the aspect thereof, any aspect of a plant food or drink product alternative to a general animal protein prepared product is mentioned.

The prepared product is not particularly limited as long as it is a food or drink product alternative to a general animal protein prepared product, and specific examples thereof include an alternative milk, an alternative meat, an alternative cheese, and an alternative yogurt.

3. Agent of Increasing Free Glutamic Acid Content

A combination of the proteolytic enzyme and the protein glutaminase can improve (increase) the free glutamic acid content in the plant protein-containing composition. Therefore, the present invention also provides an agent of increasing a free glutamic acid content of a plant protein-containing composition, the agent containing a proteolytic enzyme and a protein glutaminase.

The type, used amount, and the like of the component to be used in the agent of increasing a free glutamic acid content are as described in the section of "1. Method for Producing Processed Plant Protein-Containing Composition".

Examples

Hereinafter, the present invention will be specifically described by means of Examples; however, the present invention is not to be construed as being limited to the following Examples.

[Enzyme to Be Used]

The following enzymes (all manufactured by Amano Enzyme Inc.) were used.

(1) Proteolytic Enzyme

(1-1) Protease

- Protease derived from Aspergillus niger
- Protease derived from Rhizopus niveus
- Protease derived from Aspergillus oryzae
- Protease derived from Aspergillus melleus
- Protease derived from Bacillus licheniformis
- Protease derived from Bacillus amyloliquefaciens
- Protease derived from Geobacillus stearothermophilus

(1-2) Peptidase

- Peptidase derived from Aspergillus oryzae
- Peptidase derived from Rhizopus oryzae

(2) Protein Glutaminase

- Protein glutaminase (PG) derived from Chryseobacterium proteolyticum

(3) Glutaminase

- Glutaminase derived from Bacillus amyloliquefaciens

(4) α-Amylase

- α-Amylase derived from Bacillus amyloliquefaciens

[Plant Protein Material to Be Used]

[Table 1]

| Product name | Plant protein | Aspect | Protein content (weight basis) | Manufacturer |
|---|---|---|---|---|
| SOYPRO | Soybean protein | Powder | 50% or more | J-OIL MILLS, INC. |
| NUTRALYS F85M | Pea protein | Powder | 85% or more | Roquette Japan K.K. |
| VITEN | Wheat protein | Powder | 80% or more | Roquette Japan K.K. |
| MUNG BEAN PROTEIN | Mung bean protein | Powder | 80% or more | Organo Corporation |

(continued)

| Product name | Plant protein | Aspect | Protein content (weight basis) | Manufacturer |
|---|---|---|---|---|
| FAVA BEAN PROTEIN | Fava bean protein | Powder | 83.7% or more | Organo Corporation |
| CHICKPEA PROTEIN | Chickpea protein | Powder | 84% or more | Organo Corporation |
| OAT PROTEIN | Oat protein | Powder | 75% or more | Organo Corporation |
| CHIA SEED PROTEIN | Chia seed protein | Powder | 83% or more | Organo Corporation |
| LENTIL BEAN PROTEIN | Lentil bean protein | Powder | 50% or more | Organo Corporation |
| RICE PROTEIN | Rice protein | Powder | 40.3% or more | Bio Actives Japan Corporation |
| HEMP PROTEIN | Hemp seed ※ protein | Powder | 85% or more | Bio Actives Japan Corporation |
| ALMOND PROTEIN | Almond protein | Powder | 60% or more | Bio Actives Japan Corporation |
| ZEIN | Corn protein | Powder | 81% or more | FUJIFILM Wako Pure Chemical Corporation |
| Lupin isolate | Lupine bean protein | Powder | 85% or more | Wide Open Agriculture |
| HOKKAIDO RYE WHOLE-WHEAT FLOUR | Rye protein | Powder | $7.5 \pm 1.0\%$ | TOMIZAWA SHOU-TEN |
| ISHIBASHIKOHGYO BARLEY POWDER | Barley protein | Powder | 6.80% | Ishibashikohgyo Co., Ltd. |
| WHITE SORGHUM FLOUR | Sorghum protein | Powder | 10% | NAKANO INDUSTRY CO., LTD. |
| Daizu Lab "Soymeat" dry type (minced) | Soybean protein | Textured | 50% | Marukome Co., Ltd. |
| ※ Hemp seed is derived from industrial hemp not containing THC. | | | | |

(1) Plant Protein-Containing Composition (Liquid Plant Protein-Containing Composition) Used in Preliminary Test Example and Test Examples 1 and 2

The plant protein material (powder) and water were mixed to prepare a liquid plant protein-containing composition. The content of the plant protein material in the composition was set to 10 wt%.

(2) Plant Protein-Containing Composition (Textured Plant Protein Material Swollen with Water) Used in Preliminary Test Example and Test Examples 1, 3, and 4

To 10 g of the plant protein material (textured), 6 times the amount by weight of water was added. The mixture was left to stand still at 60°C for 60 minutes to swell the textured plant protein material, and after swelling, washing with water was performed. Thereafter, water was removed to obtain 25 g of the plant protein-containing composition (textured plant protein material swollen with water) (the content of the protein material in the plant protein-containing composition was about 40 wt%).

[Enzyme Activity Measurement Method]

(1) Proteolytic Enzyme Activity Value Measurement Method

(1-1) Protease Activity Value Measurement Method

After 5 mL of 0.6% (w/v) casein solution (0.05 mol/L sodium hydrogen phosphate, pH 8.0 [in the case of a protease derived from Geobacillus stearothermophilus, Bacillus amyloliquefaciens, Bacillus licheniformis, or Aspergillus melleus] or 0.7% (v/w) lactic acid, pH 3.0 [in the case of a protease derived from Aspergillus niger, Aspergillus oryzae, or Rhizopus niveus]) was warmed at 37°C for 10 minutes, 1 mL of a sample solution containing a protease was added, and the mixture was immediately shaken. After this solution was left to stand at 37°C for 10 minutes, 5 mL of a trichloroacetic acid reagent (containing 1.8% (w/v) trichloroacetic acid, 1.8% (w/v) sodium acetate, and 0.33 mol/L acetic acid [in the case of a protease derived from Geobacillus stearothermophilus, Bacillus amyloliquefaciens, Bacillus licheniformis, or Aspergillus melleus], or containing 0.44 mol/L trichloroacetic acid [in the case of a protease derived from Aspergillus niger, Aspergillus oryzae, or Rhizopus niveus]) was added, the mixture was shaken, and left to stand at 37°C for 30 minutes again, and the mixture was filtered. The first filtrate (3 mL) was removed, the next filtrate (2 mL) was weighed, 5 mL of a 0.55 mol/L sodium carbonate reagent and 1 mL of Folin's reagent (1 → 3) were added, and the mixture was shaken well and left to stand at 37°C for 30 minutes. An absorbance AT of this solution (enzymatic reaction solution) at a wavelength of 660 nm was measured using water as a control.

Separately, an absorbance AB of a solution (blank) obtained by performing the same operation as in the above-described enzymatic reaction solution was measured, except that 1 mL of a sample solution containing a protease was weighed, 5 mL of a trichloroacetic acid reagent (containing 1.8% (w/v) trichloroacetic acid, 1.8% (w/v) sodium acetate, and 0.33 mol/L acetic acid [in the case of a protease derived from Geobacillus stearothermophilus, Bacillus amyloliquefaciens, Bacillus licheniformis, or Aspergillus melleus] or containing 0.44 mol/L trichloroacetic acid [in the case of a protease derived from Aspergillus niger, Aspergillus oryzae, or Rhizopus niveus]) was added, the mixture was immediately shaken, 5 mL of a 0.6% (w/v) casein solution (0.05 mol/L sodium hydrogen phosphate, pH 8.0 [in the case of a protease derived from Geobacillus stearothermophilus, Bacillus amyloliquefaciens, Bacillus licheniformis, or Aspergillus melleus] or 0.7% (v/w) lactic acid, pH 3.0 [in the case of a protease derived from Aspergillus niger, Aspergillus oryzae, or Rhizopus niveus]) was then added, and the mixture was immediately shaken and left to stand at 37°C for 30 minutes.

An amount of an enzyme which causes an increase in colored materials by Folin's reagent corresponding to 1 μg of tyrosine per minute was defined as 1 unit (1 U).

Each of 1 mL, 2 mL, 3 mL, and 4 mL of a 1 mg/mL tyrosine standard stock solution (0.2 mol/L of hydrochloric acid) was weighed, and a 0.2 mol/L hydrochloric acid reagent was added thereto to make 100 mL. Each solution (2 mL) was weighed, 5 mL of a 0.55 mol/L sodium carbonate reagent and 1 mL of Folin's reagent (1 → 3) were added, and the mixture was immediately shaken and left to stand at 37°C for 30 minutes. For these solutions, absorbances A1, A2, A3, and A4 at a wavelength of 660 nm were measured using, as a control, a solution obtained by weighing 2 mL of a 0.2 mol/L hydrochloric acid reagent and performing the same operation as described above. The absorbances A1, A2, A3, and A4 were plotted on the vertical axis and the amount (μg) of tyrosine in 2 mL of each solution was plotted on the horizontal axis to prepare a calibration curve, and the amount (μg) of tyrosine with respect to the absorbance difference of 1.

[Mathematical Formula 1]

$$\text{Protease activity (U/g, U/mL)} = (AT - AB) \times F \times 11/2 \times 1/10 \times 1/M$$

AT: Absorbance of enzymatic reaction solution
AB: Absorbance of blank
F: Amount (μg) of tyrosine when difference in absorbance determined from tyrosine calibration curve is 1
11/2: Conversion factor into total solution amount after termination of reaction
1/10: Conversion factor into value per reaction time of 1 minute
M: Amount (g or mL) of sample in 1 mL of sample solution

(1-2) Peptidase Activity Value Measurement Method

As a sample solution, a solution obtained by weighing an appropriate amount of the enzyme and adding a phosphate buffer solution (0.01 mol/L) having a pH of 7.0 thereto to dissolve or uniformly disperse the enzyme and adjust the amount to 50 mL was used, or a solution obtained by further diluting the resulting solution 10 times, 100 times, or 1000 times with a buffer solution was used. As a substrate solution, a solution obtained by weighing 40 mg of L-leucyl-p-nitroanilide hydrochloride and adding a phosphate buffer solution (0.01 mol/L, containing 12.5 μmol/L of zinc sulfate) having a pH of 7.0 thereto to dissolve the L-leucyl-p-nitroanilide hydrochloride and adjust the amount to 100 mL was used.

The substrate solution (2 mL) was weighed and warmed at 37°C for 5 minutes, 0.5 mL of the sample solution was then added, and the mixture was shaken and warmed at the same temperature for 15 minutes. Thereto, 2.5 mL of 0.2 mol/L hydrochloric acid was added and mixed, and the reaction was stopped, thereby obtaining a test solution. Separately, the same operation as in the preparation of the test solution was performed using a phosphate buffer solution (0.01 mol/L) having a pH of 7.0 instead of the sample solution to prepare a comparative solution. When the absorbance at a wavelength of 405 nm is measured for the test solution and the comparative solution, the absorbance of the test solution is larger than the absorbance of the comparative solution. Note that, when there was turbidity in the test solution and the comparative solution for measuring the absorbance, centrifugation was performed, and the supernatant was measured. The activity that generates 1 μmol of p-nitroaniline per minute was defined as 1 unit (1 U).

[Mathematical Formula 2]

Peptidase activity (U/g, U/mL) = (AT - AB) × F × (1/0.5) × (1/15) × N

AT: Absorbance of enzymatic reaction solution
AB: Absorbance of enzyme blank solution
F: Amount (μmol) of p-nitroaniline when difference in absorbance determined from calibration curve is 1
0.5: Enzyme solution amount (mL)
15: Reaction time (min)
N: Dilution factor

(2) Protein Glutaminase Activity Value Measurement Method

To 1 mL of a 0.2 M phosphate buffer (pH 6.5) containing 30 mM Z-Gln-Gly, 0.1 mL of a sample solution containing a protein glutaminase was added, the mixture was left to stand at 37°C for 10 minutes, and then 1 mL of a 0.4 M TCA solution was added to stop the reaction. As a blank, to 1 mL of a 0.2 M phosphate buffer (pH 6.5) containing 30 mM Z-Gln-Gly, 1 mL of a 0.4 M TCA reagent was added, 0.1 mL of a sample solution containing a protein glutaminase was further added, and the mixture was left to stand at 37°C for 10 minutes.

The amount of ammonia generated in the reaction solution was measured for the solution obtained above using Ammonia Test Wako (FUJIFILM Wako Pure Chemical Corporation). The ammonia concentration in the reaction solution was determined from a calibration curve representing the relationship between the ammonia concentration and the absorbance (630 nm) prepared using an ammonia standard solution (ammonium chloride).

The activity of the protein glutaminase (PG) was calculated from the following formula with the amount of enzyme that produces 1 μmol of ammonia per minute being defined as 1 unit (1 U). In the formula, the reaction solution amount is 2.1, the enzyme solution amount is 0.1, and Df is a dilution rate of the enzyme solution. 17.03 is a molecular weight of ammonia.

[Mathematical Formula 3]

PG activity (U/mL)
= Ammonia concentration (mg/L) in reaction solution × (1/17.03)
× (Reaction solution amount/Enzyme solution amount) × (1/10) × Df

(3) Glutaminase Activity Value Measurement Method

As a sample solution, a solution obtained by weighing an appropriate amount of the enzyme and adding an acetic acid buffer solution (0.01 mol/L, pH 6.0, containing polyoxyethylene (10) octylphenyl ether) thereto to dissolve or uniformly disperse the enzyme and adjust the amount to 50 mL was used, or a solution obtained by further diluting the resulting solution 10 times or 100 times with the same buffer solution was used.

As a substrate solution, a solution obtained by weighing 2.0 g of L-glutamine, adding 70 mL of water to dissolve the L-glutamine, adding 10 mL of an acetic acid buffer solution (1 mol/L) having a pH of 6.0, and adding water to adjust the amount to 100 mL was used. The sample solution (1 mL) was weighed and warmed in a water bath at 37°C for 5 minutes, 1 mL of the substrate solution warmed at 37°C in advance was added thereto, the mixture was immediately shaken and further warmed at 37°C for 10 minutes, 1 mL of perchloric acid (83 → 1000) was then added, and the mixture was shaken and immediately cooled in ice water for 1 minute or more. However, perchloric acid having a mass fraction of 60% was used. To this solution, 1 mL of a sodium hydroxide solution (3 → 100) was added and shaken to obtain a test solution.

Separately, 1 mL of the sample solution was weighed, 1 mL of perchloric acid (83 → 1000) was then added thereto, the mixture was shaken and warmed in a water bath at 37°C for 5 minutes, 1 mL of the substrate solution was added, the mixture was shaken and cooled in ice water for 1 minute or more. To this solution, 1 mL of a sodium hydroxide solution (3 → 100) was added and shaken to obtain a comparative solution. When the absorbance at a wavelength of 555 nm was measured using an L-glutamic acid measuring kit (manufactured by YAMASA CORPORATION), the absorbance of the solution obtained by adding the test solution was larger than the absorbance of the solution obtained adding the comparative solution. Note that, when there was turbidity in the test solution and the comparative solution for measuring the absorbance, centrifugation was performed, and the supernatant was measured. An amount of an enzyme producing 1 μmol of L-glutamic acid per minute was defined as 1 unit (1 GTU).

Glutaminase activity (GTU/g, GTU/mL) = [(AS - ASB) · (AR - ARB)] × 250 × 4 × 1/147 × 1/10 × n                [Mathematical Formula 4]

AS: Absorbance of reaction solution
ASB: Absorbance of blank solution
AR: Absorbance of L-glutamic acid standard solution (250 mg/L)
ARB: Absorbance of water blank solution
250: L-glutamic acid standard solution concentration (mg/L)
4: Reaction solution total amount (mL)
147: Molecular weight of L-glutamic acid
10: Reaction time (min)
n: Sample dilution factor

(4) α-Amylase Activity Value Measurement Method

After 10 mL of a 1 wt% potato starch substrate solution (0.1 mol/L of acetic acid (pH 5.0)) was warmed at 37°C for 10 minutes, 1 mL of a sample solution containing an α-amylase was then added, and the mixture was immediately shaken. This solution was left to stand at 37°C for 10 minutes, 1 mL of this solution was then added to 10 mL of a 0.1 mol/L hydrochloric acid reagent, and the mixture was immediately shaken. Next, 0.5 mL of this solution was weighed, 10 mL of 0.0002 mol/L iodine reagent (the Japanese pharmacopoeia) was added, the mixture was shaken, and then an absorbance (AT) at a wavelength of 660 nm was measured using water as a control. Separately, an absorbance (AB) was measured by the same operation adding 1 mL of water instead of the sample solution. The amount of enzyme that reduces the color of potato starch with iodine by 10% per minute was defined as 1 unit (1 U).

[Mathematical Formula 5]

$$\alpha\text{-Amylase activity (U/g, U/mL)} = (AB - AT)/AB \times 1/W$$

AT: Absorbance of reaction solution
AB: Absorbance of blank solution
W: Amount (g or mL) of sample in 1 mL of sample solution

[Preliminary Test Example]

(1) Experimental Method

To 10 mL of a liquid plant protein-containing composition (10 wt% of plant protein material) (pH 6 to 7 (25°C)), 3 parts by weight of proteolytic enzyme and 0.3 parts by weight of PG per 100 parts by weight of the plant protein material were added. As a control experiment, a test fraction of only 3 parts by weight of proteolytic enzyme per 100 parts by weight of the plant protein material was prepared. The test fraction was reacted at 50°C for 2 hours by shaking at 170 rpm. After the reaction, the enzyme was deactivated by boiling and cooled to room temperature to obtain a processed plant protein-containing liquid composition.

(2) Free Amino Nitrogen (FAN) Content Measurement

The obtained processed plant protein-containing liquid composition was centrifuged at 15,000 rpm for 5 minutes to obtain a supernatant. The supernatant was diluted at a predetermined dilution ratio, and 0.5 mL of a ninhydrin reagent with pH 6.7 (10% $Na_2HPO_4/12H_2O$, 6% $KH_2PO_4$, 0.50% ninhydrin, 0.30% fructose) was added to 1.0 mL of the diluted solution. After mixing, the mixture was boiled for 16 minutes, and the absorbance at 570 nm was measured. The free amino nitrogen content obtained in the case of using a proteolytic enzyme and PG was derived as a relative value when the free amino nitrogen content obtained in the control experiment (the experiment performed in the same manner except that PG was not used) was taken as 1. The results are shown in Table 2.

(3) Experimental results

[Table 2]

| | | Reference Example 1 | Reference Example 2 | Reference Example 3 | Reference Example 4 | Reference Example 5 | Reference Example 6 | Reference Example 7 | Reference Example 8 | Reference Example 9 | Reference Example 10 | Reference Example 11 | Reference Example 12 | Reference Example 13 | Reference Example 14 | Reference Example 15 | Reference Example 16 |
|---|---|---|---|---|---|---|---|---|---|---|---|---|---|---|---|---|---|
| Used Enzyme | Protease derived from Aspergillus niger | 6,000 | 6,000 | - | - | - | - | - | - | - | - | - | - | - | - | - | - |
| | Protease derived from Rhizopus niveus | - | - | 1,200 | 1,200 | - | - | - | - | - | - | - | - | - | - | - | - |
| | Protease derived from Aspergillus oryzae | - | - | - | - | 1,200 | 1,200 | - | - | - | - | - | - | - | - | - | - |
| | Protease derived from Aspergillus melleus | - | - | - | - | - | - | 9,000 | 9,000 | - | - | - | - | - | - | - | - |
| | Protease derived from Bacillus amyloliquefaciens | - | - | - | - | - | - | - | - | 2,100 | 2,100 | - | - | - | - | - | - |
| | Protease derived from Bacillus licheniformis | - | - | - | - | - | - | - | - | - | - | 2,400 | 2,400 | - | - | - | - |
| | Protease derived from Geobacillus stearothermophilus | - | - | - | - | - | - | - | - | - | - | - | - | 2,700 | 2,700 | - | - |
| | Peptidase derived from Aspergillus oryzae | - | - | - | - | - | - | - | - | - | - | - | - | - | - | 42 | 42 |
| | PG | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| Plant protein material | Soybean | 10 wt% | - | 10 wt% | - | 10 wt% | - | 10 wt% | - | 10 wt% | - | 10 wt% | - | 10 wt% | - | 10 wt% | - |
| | Pea | - | 10 wt% | - | 10 wt% | - | 10 wt% | - | 10 wt% | - | 10 wt% | - | 10 wt% | - | 10 wt% | - | 10 wt% |
| Relative value of free amino nitrogen content (relative value when the free amino nitrogen content in the case of not using PG is taken as "1") | | 1.7 | 1.9 | 1.1 | 1.2 | 3.2 | 3.4 | 2.5 | 2.6 | 1.1 | 1.2 | 1.2 | 1.3 | 1.3 | 1.4 | 3.5 | 3.7 |

In the table, the amount of the enzyme used is represented as the activity value (U/g) per 1 g of the plant protein material.

As shown in Table 2, it was found that the relative free amino nitrogen content was increased by using the proteolytic enzyme and the protein glutaminase in combination as compared with the case of using the proteolytic enzyme alone. That is, it was found that the protein glutaminase improved the reactivity of the proteolytic enzyme. In particular, in the case of using, as the proteolytic enzyme, a protease derived from Aspergillus niger, Rhizopus niveus, Aspergillus oryzae, or Aspergillus melleus, or a peptidase derived from Aspergillus oryzae, the increase in relative free amino nitrogen content was remarkable. The increase in relative free amino nitrogen content was also observed in both soybean protein and pea protein.

Also in the case of performing the same operation by changing the liquid plant protein-containing composition to a textured plant protein material swollen with water, an increase in relative free amino nitrogen content was observed.

[Test Example 1]

(1) Experimental Method

A processed plant protein-containing liquid composition was obtained by treating the liquid plant protein-containing composition with various enzymes in the same manner as in the above (1) of Preliminary Test Example, except that each enzyme shown in Table 3 was used in an amount shown in the table (only a peptidase derived from R. oryzae was 0.3 parts by weight per 100 parts by weight of the plant protein material, other proteolytic enzymes corresponded to 3 parts by weight per 100 parts by weight of the plant protein material).

(2) Free Amino Acid Analysis

The obtained processed plant protein-containing liquid composition was centrifuged at 15,000 rpm for 5 minutes, the supernatant was filtered through a filter (0.45 μm), and then the amount of free amino acids was analyzed with an amino acid analyzer (amino acid analysis using Agilent 1260 Infinity II LC system) according to the protocol. The free glutamic acid content obtained in the case of using a proteolytic enzyme and PG was derived as a relative value when the free glutamic acid content obtained in the control experiment (the experiment performed in the same manner except that PG was not used) was taken as 1. The results are shown in Table 3.

(3) Food Texture Evaluation

The food texture (specifically, feeling on the tongue) of the obtained processed plant protein-containing liquid composition was scored by trained panelists on the basis of VAS in which the gritty feeling on the tongue of the plant protein-containing liquid composition treated in the same manner except that the enzyme treatment was not performed was rated as "1 point" and the smooth feeling on the tongue in which the gritty texture was almost not observed was rated as "10 points". The results are shown in Table 3. Note that, in Table 3, the obtained score (referred to as Score A) is arranged with the score (referred to as Score B) obtained in the control experiment (the experiment performed in the same manner except that PG was not used), and is shown in the form of Score A/Score B.

(4) Experimental results

[Table 3]

| | | Example 1 | Example 2 | Example 3 | Example 4 | Example 5 | Example 6 | Example 7 | Example 8 | Example 9 | Example 10 | Example 11 | Example 12 | Example 13 | Example 14 | Example 15 | Example 16 | Example 17 | Example 18 |
|---|---|---|---|---|---|---|---|---|---|---|---|---|---|---|---|---|---|---|---|
| Used Enzyme | Protease derived from Aspergillus niger | 6,000 | 6,000 | - | - | - | - | - | - | - | - | - | - | - | - | - | - | - | - |
| | Protease derived from Rhizopus niveus | - | - | 1,200 | 1,200 | - | - | - | - | - | - | - | - | - | - | - | - | - | - |
| | Protease derived from Aspergillus oryzae | - | - | - | - | 1,200 | 1,200 | - | - | - | - | - | - | - | - | - | - | - | - |
| | Protease derived from Aspergillus melleus | - | - | - | - | - | - | 9,000 | 9,000 | - | - | - | - | - | - | - | - | - | - |
| | Protease derived from Bacillus amyloliquefaciens | - | - | - | - | - | - | - | - | - | - | - | - | 2,100 | 2,100 | - | - | - | - |
| | Protease derived from Bacillus licheniformis | - | - | - | - | - | - | - | - | - | - | - | - | - | - | 2,400 | 2,400 | - | - |
| | Protease derived from Geobacillus stearothermophilus | - | - | - | - | - | - | - | - | - | - | - | - | - | - | - | - | 2,700 | 2,700 |
| | Peptidase derived from Aspergillus oryzae | - | - | - | - | - | - | - | - | 42 | 42 | - | - | - | - | - | - | - | - |
| | Peptidase derived from Rhizopus oryzae | - | - | - | - | - | - | - | - | - | - | 0.3 | 0.3 | 0.3 | 0.3 | 0.3 | 0.3 | 0.3 | 0.3 |
| | PG | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| Plant protein material | Soybean | 10 wt% | - | 10 wt% | - | 10 wt% | - | 10 wt% | - | 10 wt% | - | 10 wt% | - | 10 wt% | - | 10 wt% | - | 10 wt% | - |
| | Pea | - | 10 wt% | - | 10 wt% | - | 10 wt% | - | 10 wt% | - | 10 wt% | - | 10 wt% | - | 10 wt% | - | 10 wt% | - | 10 wt% |
| | Free glutamic acid content relative value (relative value when the free glutamic acid content in the case of not using PG is taken as "1") | 1.69 | 1.12 | 1.33 | 1.23 | 1.57 | 1.3 | 1.3 | 1.19 | 1.48 | 1.43 | 2.65 | 2.38 | 2.62 | 2.26 | 4.85 | 2.19 | 3.28 | 2.28 |
| | Food texture improvement score (score according to Examples/score in case of not using PG) | 8/4 | 6/3 | 7/5 | 5/2 | 10/5 | 9/5 | 10/5 | 9/4 | 10/5 | 9/4 | / | / | 7/4 | 6/3 | 7/4 | 4/2 | 7/6 | 7/3 |

In the table, the amount of the enzyme used is represented as the activity value (U/g) per 1 g of the plant protein material.

As shown in Table 3, it was observed that the relative free glutamic acid content was increased by using the proteolytic enzyme and the protein glutaminase in combination as compared with the case of using the proteolytic enzyme alone. In particular, in the case of using a peptidase derived from Rhizopus oryzae, the increase in relative free glutamic acid content was especially remarkable. The increase in relative free glutamic acid content was also observed in both soybean protein and pea protein. Note that, the processed plant protein-containing liquid composition obtained in each of Examples was subjected to sensory evaluation of taste by trained panelists, and as a result, it was found that bitterness was reduced in all Examples as compared with the case of not using PG.

As shown in Table 3, the effect of improving the food texture of the processed plant protein-containing liquid composition was also observed by using the proteolytic enzyme and the protein glutaminase in combination as compared with the case of using the proteolytic enzyme alone.

Also in the case of performing the same operation by changing the liquid plant protein-containing composition to a textured plant protein material swollen with water, an increase in relative free glutamic acid content was observed.

[Test Example 2]

A processed plant protein-containing liquid composition was obtained in the same manner as in (1) of Test Example 1, except that the combination of the enzyme to be used and the plant protein material was changed to those shown in Tables 4 and 5, and regarding Examples 31 to 33 and 46 to 48, the plant protein material was changed to one subjected to a pre-treatment with α-amylase (specifically, α-amylase in an amount of 6.5 U per 1 g of the plant protein material was added to a liquid plant protein-containing composition (10 wt% of the plant protein material), and the mixture was treated at 70°C for 0.5 hours and cooled to 50°C).

For the obtained processed plant protein-containing liquid composition, the free glutamic acid content (absolute value) was measured in the same manner as in (2) of Test Example 1, and the relative value of the free glutamic acid content was derived. The results are shown in Tables 4 and 5.

Note that, among Examples shown in Tables 4 and 5, the relative value and the absolute value of the free glutamic acid content of Examples 24 and 39 were compared with those of Comparative Examples (Comparative Examples 1 and 3) which were processed plant protein-containing liquid compositions obtained by performing the treatment in the same manner except that a protein glutaminase was not used and those of Comparative Examples (Comparative Examples 2 and 4) which were processed plant protein-containing liquid compositions obtained by performing the treatment in the same manner except that a glutaminase was used instead of a protein glutaminase. The results are shown in Table 6.

[Table 4]

| | | Example 19 | Example 20 | Example 21 | Example 22 | Example 23 | Example 24 | Example 25 | Example 26 | Example 27 | Example 28 | Example 29 | Example 30 | Example 31 | Example 32 | Example 33 |
|---|---|---|---|---|---|---|---|---|---|---|---|---|---|---|---|---|
| Used Enzyme | Protease derived from Aspergillus oryzae | 1,200 | 1,200 | 1,200 | 1,200 | 1,200 | 1,200 | 1,200 | 1,200 | 1,200 | 1,200 | 1,200 | 1,200 | 1,200 | 1,200 | 1,200 |
| | PG | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| Plant protein material | Fava bean | 10 wt% | - | - | - | - | - | - | - | - | - | - | - | - | - | - |
| | Mung bean | - | 10 wt% | - | - | - | - | - | - | - | - | - | - | - | - | - |
| | Chickpea | - | - | 10 wt% | - | - | - | - | - | - | - | - | - | - | - | - |
| | Lentil bean | - | - | - | 10 wt% | - | - | - | - | - | - | - | - | - | - | - |
| | Lupine bean | - | - | - | - | 10 wt% | - | - | - | - | - | - | - | - | - | - |
| | Gluten (wheat) | - | - | - | - | - | 10 wt% | - | - | - | - | - | - | - | - | - |
| | Oat | - | - | - | - | - | - | 10 wt% | - | - | - | - | - | - | - | - |
| | Rice | - | - | - | - | - | - | - | 10 wt% | - | - | - | - | - | - | - |
| | ZEIN (corn) | - | - | - | - | - | - | - | - | 10 wt% | - | - | - | - | - | - |
| | Almond | - | - | - | - | - | - | - | - | - | 10 wt% | - | - | - | - | - |
| | Hemp seed | - | - | - | - | - | - | - | - | - | - | 10 wt% | - | - | - | - |
| | Chia seed | - | - | - | - | - | - | - | - | - | - | - | 10 wt% | - | - | - |
| | Rye | - | - | - | - | - | - | - | - | - | - | - | - | 10 wt% | - | - |
| | Barley | - | - | - | - | - | - | - | - | - | - | - | - | - | 10 wt% | - |
| | Sorghum | - | - | - | - | - | - | - | - | - | - | - | - | - | - | 10 wt% |
| Free glutamic acid content relative value (relative value when the free glutamic acid content in the case of not using PG is taken as "1") | | 1.23 | 1.18 | 2.04 | 1.57 | 1.46 | 9.02 | 5.14 | 1.87 | 9.96 | 1.87 | 1.47 | 1.37 | 2.05 | 1.92 | 3.17 |

In the table, the amount of the enzyme used is represented as the activity value (U/g) per 1 g of the plant protein material.

[Table 5]

| | | Example 34 | Example 35 | Example 36 | Example 37 | Example 38 | Example 39 | Example 40 | Example 41 | Example 42 | Example 43 | Example 44 | Example 45 | Example 46 | Example 47 | Example 48 |
|---|---|---|---|---|---|---|---|---|---|---|---|---|---|---|---|---|
| Used Enzyme | Peptidase derived from Aspergillus oryzae | 42 | 42 | 42 | 42 | 42 | 42 | 42 | 42 | 42 | 42 | 42 | 42 | 42 | 42 | 42 |
| | PG | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| Plant protein material | Fava bean | 10 wt% | - | - | - | - | - | - | - | - | - | - | - | - | - | - |
| | Mung bean | - | 10 wt% | - | - | - | - | - | - | - | - | - | - | - | - | - |
| | Chickpea | - | - | 10 wt% | - | - | - | - | - | - | - | - | - | - | - | - |
| | Lentil bean | - | - | - | 10 wt% | - | - | - | - | - | - | - | - | - | - | - |
| | Lupine bean | - | - | - | - | 10 wt% | - | - | - | - | - | - | - | - | - | - |
| | Gluten (wheat) | - | - | - | - | - | 10 wt% | - | - | - | - | - | - | - | - | - |
| | Oat | - | - | - | - | - | - | 10 wt% | - | - | - | - | - | - | - | - |
| | Rice | - | - | - | - | - | - | - | 10 wt% | - | - | - | - | - | - | - |
| | ZEIN (corn) | - | - | - | - | - | - | - | - | 10 wt% | - | - | - | - | - | - |
| | Almond | - | - | - | - | - | - | - | - | - | 10 wt% | - | - | - | - | - |
| | Hemp seed | - | - | - | - | - | - | - | - | - | - | 10 wt% | - | - | - | - |
| | Chia seed | - | - | - | - | - | - | - | - | - | - | - | 10 wt% | - | - | - |
| | Rye | - | - | - | - | - | - | - | - | - | - | - | - | 10 wt% | - | - |
| | Barley | - | - | - | - | - | - | - | - | - | - | - | - | - | 10 wt% | - |
| | Sorghum | - | - | - | - | - | - | - | - | - | - | - | - | - | - | 10 wt% |
| Free glutamic acid content relative value (relative value when the free glutamic acid content in the case of not using PG is taken as "1") | | 1.36 | 1.22 | 1.9 | 1.36 | 1.65 | 9.83 | 4.19 | 2.07 | 8.89 | 2.07 | 1.66 | 1.5 | 2.01 | 2.27 | 3.03 |

In the table, the amount of the enzyme used is represented as the activity value (U/g) per 1 g of the plant protein material.

[Table 6]

| | | Comparative Example 1 | Comparative Example 2 | Example 24 | Comparative Example 3 | Comparative Example 4 | Example 39 |
|---|---|---|---|---|---|---|---|
| Used Enzyme | Peptidase derived from Aspergillus oryzae | 1,200 U/g | 1,200 U/g | 1,200 U/g | - | - | - |
| | Peptidase derived from Aspergillus oryzae | - | - | - | 42 U/g | 42 U/g | 42 U/g |
| | Glutaminase derived from Bacillus amyloliquefaciens | - | 0.3 GTU/g | - | - | 0.3 GTU/g | - |
| | PG | - | - | 1.5 U/g | - | - | 1.5 U/g |
| Plant protein material | Gluten (wheat) | 10 wt% | 10 wt% | 10 wt% | 10 wt% | 10 wt% | 10 wt% |
| Free glutamic acid content relative value (relative value when the free glutamic acid content in the case of not using PG and glutaminase is taken as "1") | | 1 | 5.13 | 9.02 | 1 | 5.84 | 9.83 |
| Free glutamic acid content absolute value | | 0.12 mM | 0.62 mM | 1.08 mM | 0.16 mM | 0.93 mM | 1.57 mM |
| In the table, the amount of the enzyme used is represented as the activity value (U/g) per 1 g of the plant protein material. | | | | | | | |

As shown in Tables 4 and 5, the increase in free glutamic acid content was observed in all the plant protein materials. As shown in Table 6, when the plant protein material was treated using the proteolytic enzyme and the protein glutaminase in combination, the free glutamic acid content in the processed plant protein material was remarkably increased, and the degree thereof was sufficient to not only reduce the bitterness of the processed plant protein-containing liquid composition but also impart umami.

[Test Example 3]

With 25 g of the textured plant protein material swollen with water, 10 mL of water, 5 mL of olive oil, methyl cellulose (final concentration: 2 mass%), and the enzyme described in Table 7 in an amount shown in the table were mixed, and the mixture was molded into a patty shape and incubated at 50°C for 1 hour to obtain a textured plant protein-containing food product (after seasoning, before baking).

The textured plant protein-containing food product (after seasoning, before baking) was baked at 150°C for 10 minutes to obtain a patty-shaped textured plant protein-containing food product (after baking). The obtained food product was subjected to umami measurement by a taste sensor (manufactured by Intelligent Sensor Technology, Inc.) and sensory evaluation by six trained panelists to obtain umami score. **In** the sensory evaluation, the degree of umami was scored on the basis of the following classification, and the average value was taken as umami score. The results are shown in Table 7.

2 points: Umami was considerably stronger than the food product of Comparative Example 5.
1 point: Umami was slightly stronger than the food product of Comparative Example 5.
0 point: Umami was equal to the food product of Comparative Example 5.
-1 point: Umami was slightly weaker than the food product of Comparative Example 5.
-2 points: Umami was considerably weaker than the food product of Comparative Example 5.

[Table 7]

|  |  | Comparative Example 5 | Comparative Example 6 | Comparative Example 7 | Comparative Example 8 | Example 40 |
|---|---|---|---|---|---|---|
| Used Enzyme | Peptidase derived from Aspergillus oryzae | - | 16.8 U/g | - |  | 16.8 U/g |
|  | Glutaminase derived from Bacillus amyloliquefaciens | - | - | 0.12 GTU/g | - | - |
|  | PG | - | - | - | 0.6 U/g | 0.6 U/g |
| Umami score (taste sensor) |  |  | 0.34 | -0.12 | 0.08 | 1.87 |
| Umami score (sensory evaluation) |  | 0 | 0.4 | 0 | 0 | 1.8 |
| In the table, the amount of the enzyme used is represented as the activity value per 1 g of the textured plant protein material swollen with water. | | | | | | |

As shown in Table 7, since the umami of the food product (Example 40) obtained by treating the plant protein material using the proteolytic enzyme and the protein glutaminase in combination was increased, it can be inferred that the free glutamic acid content was remarkably increased in the food product.

[Test Example 4]

With 25 g of the textured plant protein material swollen with water, 10 mL of water, 5 mL of olive oil, methyl cellulose (final concentration: 2 mass%), and the enzyme described in Table 8 in an amount shown in the table were mixed, and the mixture was molded into a patty shape and incubated at 50°C for 1 hour to obtain a textured plant protein-containing food product (after seasoning, before baking).

The textured plant protein-containing food product (after seasoning, before baking) was baked at 150°C for 10 minutes to obtain a patty-shaped textured plant protein-containing food product (after baking). The obtained food product was subjected to sensory evaluation by six trained panelists to obtain a bitterness score. In the sensory evaluation, the degree of bitterness was scored on the basis of the following classification, and the average value was taken as a bitterness score. The results are shown in Table 8.

2 points: Bitterness was considerably stronger than the food product of Comparative Example 5.
1 point: Bitterness was slightly stronger than the food product of Comparative Example 5.
0 point: Bitterness was equal to the food product of Comparative Example 5.
-1 point: Bitterness was slightly weaker than the food product of Comparative Example 5.
-2 points: Bitterness was considerably weaker than the food product of Comparative Example 5.

[Table 8]

|  |  | Comparative Example 5 | Comparative Example 6 | Comparative Example 9 | Example 40 |
|---|---|---|---|---|---|
| Used Enzyme | Peptidase derived from Aspergillus oryzae | - | 16.8 U/g | 16.8 U/g | 16.8 U/g |
|  | Glutaminase derived from Bacillus amyloliquefaciens | - | - | 0.12 GTU/g | - |
|  | PG | - | - | - | 0.6 U/g |
| Bitterness score (sensory evaluation) |  | 0 | 1.6 | 1.0 | 0.4 |
| In the table, the amount of the enzyme used is represented as the activity value per 1 g of the textured plant protein material swollen with water. | | | | | |

As shown in Table 8, since the bitterness of the food product (Example 40) obtained by treating the plant protein

**EP 4 523 546 A1**

material using the proteolytic enzyme and the protein glutaminase in combination was suppressed, it can be inferred that the increase in free glutamic acid content in the food product was so remarkable that occurrence of bitterness due to hydrolysis could be effectively suppressed.

**Claims**

1. A method for producing a processed plant protein-containing composition, the production method comprising a step of treating a plant protein-containing composition with a proteolytic enzyme and a protein glutaminase.

2. The production method according to claim 1, wherein the proteolytic enzyme is a protease and/or a peptidase.

3. The production method according to claim 2, wherein the protease is used in an amount of 110 U or more per 1 g of the plant protein.

4. The production method according to claim 2, wherein the protease is a filamentous fungus-derived protease.

5. The production method according to claim 2, wherein the protease is a protease derived from the genus Aspergillus and/or the genus Rhizopus.

6. The production method according to claim 2, wherein the protease is selected from the group consisting of proteases derived from Aspergillus oryzae, Aspergillus niger, Aspergillus melleus, and Rhizopus niveus.

7. The production method according to claim 2, wherein the protease is a bacteria-derived protease.

8. The production method according to claim 7, wherein the bacteria-derived protease is a protease derived from genus Bacillus and/or genus Geobacillus.

9. The production method according to claim 7, wherein the bacteria-derived protease is selected from the group consisting of proteases derived from Bacillus stearothermophilus, Bacillus licheniformis, Bacillus amyloliquefaciens, and corresponding genus Geobacillus.

10. The production method according to claim 2, wherein the proteolytic enzyme is a bacteria-derived protease and a filamentous fungus-derived peptidase.

11. The production method according to claim 2, wherein the peptidase is a peptidase derived from the genus Rhizopus and/or the genus Aspergillus.

12. The production method according to claim 1, wherein the plant protein is a protein of a plant selected from the group consisting of beans, cereals, nuts, and seeds.

13. The production method according to claim 1, wherein the plant protein-containing composition is a liquid.

14. The production method according to claim 1, wherein the plant protein-containing composition is a textured plant protein material swollen with water.

15. An agent of increasing a free glutamic acid content of a plant protein-containing composition, the agent comprising a proteolytic enzyme and a protein glutaminase.

16. A plant protein-containing food or drink product obtained by the production method according to claim 1.

## INTERNATIONAL SEARCH REPORT

| International application No. |
| --- |
| **PCT/JP2023/018009** |

### A. CLASSIFICATION OF SUBJECT MATTER

*A23J 3/34*(2006.01)i; *A23J 3/14*(2006.01)i; *A23L 33/185*(2016.01)i; *C12P 21/06*(2006.01)i
FI:     A23J3/34; A23J3/14; C12P21/06; A23L33/185

According to International Patent Classification (IPC) or to both national classification and IPC

### B. FIELDS SEARCHED

Minimum documentation searched (classification system followed by classification symbols)

A23J3/34; A23J3/14; A23L33/185; C12P21/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); FSTA/CAplus/AGRICOLA/BIOSIS/MEDLINE/EMBASE (STN)

### C. DOCUMENTS CONSIDERED TO BE RELEVANT

| Category* | Citation of document, with indication, where appropriate, of the relevant passages | Relevant to claim No. |
| --- | --- | --- |
| X | WO 2021/201277 A1 (AMANO ENZYME INC.) 07 October 2021 (2021-10-07) examples, paragraph [0013] | 1-16 |
| X | WO 2021/251344 A1 (AMANO ENZYME INC.) 16 December 2021 (2021-12-16) claims, paragraph [0018] | 1-16 |
| X | WO 2022/014542 A1 (AMANO ENZYME INC.) 20 January 2022 (2022-01-20) examples, paragraph [0011] | 1-16 |
| P, X | WO 2022/102723 A1 (AMANO ENZYME INC.) 19 May 2022 (2022-05-19) examples, claims | 1-16 |
| P, X | WO 2022/215688 A1 (AMANO ENZYME EUROPE LTD) 13 October 2022 (2022-10-13) examples, claims | 1-16 |

☐ Further documents are listed in the continuation of Box C.          ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **19 June 2023** | **04 July 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2023/018009**

| Patent document cited in search report | | | Publication date (day/month/year) | Patent family member(s) | Publication date (day/month/year) |
|---|---|---|---|---|---|
| WO | 2021/201277 | A1 | 07 October 2021 | EP 4130286 A1 examples, paragraph [0013] CN 115380116 A | |
| WO | 2021/251344 | A1 | 16 December 2021 | (Family: none) | |
| WO | 2022/014542 | A1 | 20 January 2022 | CN 115715156 A examples, paragraph [0011] | |
| WO | 2022/102723 | A1 | 19 May 2022 | (Family: none) | |
| WO | 2022/215688 | A1 | 13 October 2022 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000050887 A
- JP 2001218590 A
- WO 2006075772 A

**Non-patent literature cited in the description**

- **HABIBI-NAJAFI, M. B.** ; **LEE, B. H**. *Bitterness in cheese. Crit. Rev. Food Sci. Nutr.*, 1996, vol. 36, 397-411
- **HIDEYUKI SUZUKI** ; **YUKO NAKAFUJI** ; **TOMOKI TAMURA**. New Preparation Method of Richness Taste Seasoning γ-Glutamylation of Protein Hydrolysate Obtained by Protease Treatment. *Soy Protein Research*, 2014, vol. 17, 42-45